Europäisches Patentamt

European Patent Office

Office européen des brevets

(11) Veröffentlichungsnummer: **0 188 725**
**A1**

# EUROPÄISCHE PATENTANMELDUNG

(21) Anmeldenummer: 85115679.4

(22) Anmeldetag: 10.12.85

(51) Int. Cl.⁴: **C 12 P 7/64**, C 11 C 3/02,
C 11 C 3/06

(30) Priorität: 22.12.84 DE 3447024

(43) Veröffentlichungstag der Anmeldung: 30.07.86
Patentblatt 86/31

(84) Benannte Vertragsstaaten: **AT BE CH DE FR GB IT LI LU NL SE**

(71) Anmelder: **HOECHST AKTIENGESELLSCHAFT,
Postfach 80 03 20, D-6230 Frankfurt am Main 80 (DE)**

(72) Erfinder: **Wullbrandt, Dieter, Dr., Bienerstrasse 29,
D-6238 Hofheim am Taunus (DE)**
Erfinder: **Keller, Reinhold, Dr., Wiesenweg 5, D-6232 Bad
Soden am Taunus (DE)**
Erfinder: **Schlingmann, Merten, Dr., Schneidhainer
Strasse 32a, D-6240 Königstein/Taunus (DE)**

(54) Verfahren zur Herstellung von Partialestern des Glycerins und kondensierter Glycerine mit Fettsäuren.

(57) Partialester des Glycerins und kondensierter Glycerine mit Fettsäuren mit 2 bis 18 Kohlenstoffatomen werden durch enzymatische Veresterung mit einer Lipase aus Candida cylindracea oder aus Rhizopus arrhizus in Gegenwart von 5 bis 20 Gew.-% Wasser, bezogen auf die Reaktionsteilnehmer, erhalten. Wenn man die Umsetzung in Gegenwart eines fetten Öls oder Mineralöls durchführt, erhält man ein Veresterungsgemisch, das als Emulgatorsystem geeignet ist.

EP 0 188 725 A1

Verfahren zur Herstellung von Partialestern des Glycerins und kondensierter Glycerine mit Fettsäuren

Partialester des Glycerins und kondensierter Glycerine mit Fettsäuren werden als Emulgatoren bzw. in Emulgatorsystemen nicht nur für technische Zwecke wie in der Textilindustrie, für Insektizidzubereitungen und Rostverhütungsmittel, sondern auch für Lebensmittel, kosmetische und pharmazeutische Zwecke eingesetzt (US-Patentschrift 3 637 774). Diese Ester wurden durch direkte Veresterung oder Umesterung hergestellt. Es ist auch bekannt, daß man – in Umkehrung zur enzymatischen Esterspaltung – mit Hilfe von Enzymen Ester herstellen kann. Diese Verfahren haben jedoch noch keinen Eingang in die Technik gefunden.

Es wurde nun ein Verfahren zur Herstellung von Partialestern des Glycerins und kondensierter Glycerine mit Fettsäuren mit 2 bis 18 Kohlenstoffatomen gefunden, das dadurch gekennzeichnet ist, daß man die Reaktionsteilnehmer in Gegenwart von 5 bis 20 Gew.-% Wasser, bezogen auf die Reaktanten, bei einer Temperatur von etwa 10 bis etwa 40°C mit einer Lipase aus Candida cylindracea oder aus Rhizopus arrhizus in Kontakt bringt. Bevorzugte Ausgestaltungen dieser Erfindung werden im folgenden näher erläutert.

Neben Glycerin werden als Alkoholkomponente die niedrigen Glieder der kondensierten Glycerine bevorzugt. Die Herstellung kondensierter Glycerine ist außer in der vorstehend genannten US-Patentschrift auch in den US-Patentschriften 2 487 208 und 3 968 169 beschrieben.

Als Fettsäuren werden die handelsüblichen Verbindungen und Gemische bevorzugt. Die Auswahl richtet sich selbstver-

0188725

ständlich nach dem Einsatzzweck: Wenn beispielsweise Emulgatoren mit einer guten Lagerfähigkeit gefordert werden, wird man gesättigte Fettsäuren vorziehen.

Der Wassergehalt des Reaktionssystems liegt vorteilhaft bei 8 bis 10 %. Die Wahl der Reaktionstemperatur richtet sich nach dem gewünschten Produkt: Wenn ein hoher Gehalt an Monoester erwünscht ist, arbeitet man vorteilhaft bei niedriger Temperatur, wobei man längere Reaktionszeiten und niedrigere Umsätze in Kauf nimmt. Höhere Durchsätze erhält man in einem Temperaturbereich von etwa 30 bis 35°C, wobei jedoch zu einem größeren Anteil eine vollständige Veresterung stattfindet, was im allgemeinen unerwünscht ist. Der Umsatz kann in gewissen Grenzen auch durch Zugabe von mehr Enzym erhöht werden.

Die erfindungsgemäß eingesetzten Lipasen sind handelsüblich und werden von einer Reihe von Firmen vertrieben. Die Enzyme werden beispielsweise auch in den folgenden Patentdokumenten erwähnt: Britische Patentschrift 1 577 933, europäische Patentanmeldung mit der Veröffentlichungsnummer 35 883, US-Patentschrift 3 275 011 (C. cylindracea) und deutsche Patentschrift 1 642 654 (R. arrhizus).

Eine besonders bevorzugte Ausgestaltung der Erfindung besteht darin, daß man die enzymatische Veresterung in Gegenwart eines fetten Öls oder Mineralöls durchführt, wobei unmittelbar ein verwendungsfähiges Emulgatorsystem erhalten wird, das beispielsweise für kosmetische oder pharmazeutische Zubereitungen geeignet ist. Es können auf diese Weise inerte, emulgierbare Stoffe direkt in eine Emulsion überführt werden, die entweder unmittelbar zur Herstellung einer der genannten Zubereitungen dienen kann, oder aber nach Mischen bzw. Verdünnen mit anderen Stoffen als Emulgator dient.

Das erfindungsgemäße Verfahren kann auch in großem Maßstab ausgeführt werden, ohne daß Probleme beim Rühren oder anderweitigen Durchmischen auftreten. Das Verfahren eignet sich somit zur technischen Durchführung, wobei vorteilhaft die Enzyme in fixierter Form eingesetzt werden.

Das Glycerin bzw. das kondensierte Glycerin wird zweckmäßig im Überschuß eingesetzt, vor allem dann, wenn niedrig veresterte Produkte erwünscht sind. Gewünschtenfalls läßt sich aus dem Produkt der Glycerinüberschuß leicht entfernen, beispielsweise durch Auswaschen mit Wasser. Wenn im Produkt ein Restgehalt an Fettsäuren stört, so können diese beispielsweise nach dem Verfahren der deutschen Auslegeschrift 1 165 192 durch Extraktion mit Lipoide lösenden Lösemitteln bei annähernd neutralem bis saurem pH-Wert, insbesondere bei schwach saurem pH-Wert, weitgehend oder vollständig entfernt werden.

Die als Nebenprodukte entstehenden total veresterten Alkohole, im Falle des Glycerins also Triglyceride, stören im allgemeinen nicht, da sie häufig ohnehin in den Emulgatorsystemen vorkommen. Es wurde bereits vorstehend erwähnt, daß eine bevorzugte Ausgestaltung der Erfindung darin besteht, daß man das Verfahren in Gegenwart von fetten Ölen durchführt, wobei man unmittelbar zu einem Emulgatorsystem gelangt.

In den folgenden Beispielen wird die Erfindung näher erläutert. Prozentangaben beziehen sich hierbei auf das Gewicht, wenn keine anderen Angaben gemacht sind.

1 "Einheit" der Enzymaktivität bezeichnet die Menge, die pro Minute bei pH 7,0 aus einem Triglycerid 1,0 Mikroäquivalent Fettsäure hydrolytisch abspaltet.

Beispiel 1

50 g Ölsäure, 59 g Glycerin, 7,6 g Wasser und 1 g kommerzielle Lipase aus Candida cylindracea (23-46 Einheiten pro mg) wurden in einem geschlossenen Gefäß bei 20°C bzw. 4°C gerührt. Nach 24 Stunden wurde die erhaltene Mischung mit 100 ml Diethylether versetzt und zur Entfernung des Glycerins mit Wasser extrahiert. Das Lösemittel wurde durch Abdampfen entfernt und ein Teil des Rückstandes zur Produktanalyse an Kieselgel S (0,063-0,2 mm, Riedel-de Haen AG) mit Chloroform-Aceton-Eisessig (96:4:0,5 Volumina) als mobile Phase aufgetrennt.

Die prozentuale Veresterung wurde durch Titration der Säure mit 0,1 N Natronlauge gegen Phenolphthalein bestimmt. Die Ergebnisse sind in Tabelle 1 angegeben.

Beispiel 2

Analog Beispiel 1 wurde Glycerin mit Ölsäure bei 20°C durch Zugabe von 0,5 g Lipase aus Candida cylindracea verestert. Die Produktzusammensetzung bezüglich der gebildeten Glyceride und der Umsatz sind in Tabelle 1 wiedergegeben.

Tabelle 1

| Beispiel | Temp.°C | TG % | DG % | MG % | Umsatz % |
|----------|---------|------|------|------|----------|
| 1 | 20 | 26 | 54 | 20 | 74 |
| 1 | 4 | 26 | 48 | 26 | 64 |
| 2 | 20 | 17 | 51 | 31 | 69 |

TG = Triglycerid; DG = Diglycerid; MG = Monoglycerid

Beispiel 3

Entsprechend Beispiel 1 wurde Laurinsäure bzw. Palmitinsäure mit Glycerin bei einem Wassergehalt von 9 % mit Lipase aus Candida cylindracea umgesetzt. Nach 20-stündiger
Reaktionszeit betrug der Umsatz 77 % bzw. 36 %. Der kleinere Veresterungsgrad im Fall der Palmitinsäure wird durch
eine geringere Triglyceridbildung kompensiert.

Beispiel 4

60 g Ölsäure, 54 g Glycerin, 10 g Wasser und 0,1 g Lipase
aus Rhizopus arrhizus (1200 Einheiten pro mg, Calbiochem
GmbH) wurden bei 20°C umgesetzt. Bei 54-prozentigem Umsatz
(20 h) wurde die Reaktionsmischung entsprechend Beispiel 1
analysiert. Die prozentuale Zusammensetzung der Glyceride
ergab sich zu 18 % TG, 73 % DG und 7 % MG.

Beispiel 5

30 g Diglycerin und 25 g Ölsäure wurden unter Zusatz von
3,8 g Wasser mit 0,6 g Lipase aus Candida cylindracea
verestert. Nach 24-stündigem Rühren bei 20°C und einem Umsatz von 45 % wurde die Reaktionsmischung zur Desaktivierung der Lipase 1 Stunde auf 80°C erwärmt.

Produktanalyse: Hydroxylzahl    845
                Säurezahl        45

Beispiel 6

Zur Herstellung eines Fertigemulgatorsystems wurde analog
Beispiel 1 Glycerin mit Ölsäure unter Zusatz von 20 Gewichtsprozent Paraffin durch Zugabe der Lipase aus Candida
cylindracea bei Raumtemperatur verestert. Der Umsatz an Fettsäure sowie die Zusammensetzung der gebildeten Glyceride ist analog den Ergebnissen aus Beispiel 2.

Beispiel 7

200 g Ölsäure, 300 g Glycerin und 50 g Wasser wurden unter Zugabe von 4 g Lipase aus Candida cylindracea umgesetzt. Nach 24-stündigem Rühren bei 20°C betrug der Umsatz 65 %. Die Reaktionsmischung wurde analog Beispiel 1 aufgearbeitet und die Produktzusammensetzung chromatographisch bestimmt.

Die Ölsäure wurde anschließend durch Vakuumdestillation (0,25 mbar, 140-160°C) entfernt.

In Tabelle 2 sind die prozentualen Anteile der gebildeten Glyceride vor und nach der Destillation aufgelistet.

Tabelle 2

| % | vor | nach |
|---|---|---|
| | Destillation | |
| TG | 26 | 52 |
| DG | 54 | 31 |
| MG | 20 | 17 |

Die erhaltenen Werte weisen auf eine intermolekulare Umesterung während der Fettsäuredestillation hin.

## PATENTANSPRÜCHE

1. Verfahren zur Herstellung von Partialestern des Glycerins und kondensierter Glycerine mit Fettsäuren mit 2 bis 18 Kohlenstoffatomen, dadurch gekennzeichnet, daß man die Reaktionsteilnehmer in Gegenwart von 5 bis 20 Gew.-% Wasser, bezogen auf die Reaktanten, bei einer Temperatur von etwa 10 bis etwa 40°C mit einer Lipase aus Candida cylindracea oder Rhizopus arrhizus in Kontakt bringt.

2. Verfahren nach Anspruch 1, dadurch gekennzeichnet, daß der Wassergehalt 8 bis 10 % beträgt.

3. Verfahren nach Anspruch 1 oder 2, dadurch gekennzeichnet, daß man die Glycerinkomponente im Überschuß einsetzt.

4. Verfahren nach einem oder mehreren der vorhergehenden Ansprüche, dadurch gekennzeichnet, daß man die Umsetzung in Gegenwart eines fetten Öls oder Mineralöls durchführt.

5. Verwendung des Produktes nach Anspruch 4 als Emulgatorsystem.

# EUROPÄISCHER RECHERCHENBERICHT

Europäisches Patentamt

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 101, Nr. 15, Oktober 1984, Seite 573, Nr. 128870e, Columbus, Ohio, US; & JP - A - 59 118 094 (NIPPON OILS AND FATS CO., LTD.) 07.07.1984 * Zusammenfassung * ----- | 1-3 | C 12 P 7/64 C 11 C 3/02 C 11 C 3/06 |

RECHERCHIERTE SACHGEBIETE (Int. Cl.4)

C 12 P

Der vorliegende Recherchenbericht wurde für alle Patentansprüche erstellt.

| Recherchenort DEN HAAG | Abschlußdatum der Recherche 24-04-1986 | Prüfer PEETERS J.C. |
|---|---|---|